# EUROPÄISCHE PATENTSCHRIFT

(11) **EP 3 368 895 B1**
(45) Veröffentlichungstag und Bekanntmachung des Hinweises auf die Patenterteilung: **11.08.2021**
(21) Anmeldenummer: 16809625.3
(22) Anmeldetag: 31.10.2016
(51) Int. Cl.: G01N 33/18, G01N 1/38

(54) **PROBENVERDÜNNUNG**
SAMPLE DILUTION
DILUTION D'ÉCHANTILLON

(30) Priorität: 30.10.2015 DE 102015118586
(43) Veröffentlichungstag der Anmeldung: 05.09.2018
(60) Teilanmeldung: 21178487.1
(73) Patentinhaber: LAR Process Analysers AG, 12057 Berlin (DE)
(72) Erfinder: ARTS, Werner, 10825 Berlin (DE); ARTS, Olivia, 12355 Berlin (DE); GLITTENBERG, Martin, 42289 Wuppertal (DE)
(74) Vertreter: Heinze, Ekkehard
(86) Internationale Anmeldenummer: PCT/DE2016/100511
(87) Internationale Veröffentlichungsnummer: WO 2017/071696

(56) Entgegenhaltungen:
- EP-A1- 2 662 690
- EP-A2- 1 022 564
- US-A- 4 277 438
- US-A- 4 968 485
- US-B2- 8 101 417

## Beschreibung

Die Erfindung betrifft ein Probenanalysegerät zur Analyse einer Probelösung, insbesondere von verunreinigtem Wasser oder von Abwasser, mit einem Reaktionsgefäß zum thermischen Aufschluss einer abgemessenen Probe der zu analysierenden Probelösung, wobei das Reaktionsgefäß einen Injektionsport zum Eintragen der Probe in das Reaktionsgefäß aufweist, mindestens einem Probelösungs-Speichergefäß zur geräteinternen Speicherung von Probelösung und einer zwischen dem Probelösungs-Speichergefäß und dem Reaktionsgefäß verfahrbaren und zum Aufnehmen der Probe aus dem Probelösungs-Speichergefäß und zum Eintragen der Probe in den Injektionsport des Reaktionsgefäßes steuerbaren Injektionsspritzeneinrichtung.

Sie betrifft des Weiteren ein Verfahren zur Analyse einer Probelösung, insbesondere von verunreinigtem Wasser oder von Abwasser, wobei Probelösung geräteintern in mindestens einem Probelösungs-Speichergefäß gespeichert wird und eine mengenmäßig vorbestimmte Probe der Probelösung in einem Reaktionsgefäß thermisch aufgeschlossen wird und die Aufschlussprodukte einer Detektionseinrichtung zur quantitativen Detektion von Elementen, insbesondere Kohlenstoff, Stickstoff oder Phosphor zugeführt werden.

Es ist bekannt, wässrige Lösungen, insbesondere Abwasser oder auch Frischwasser, mit dem Ziel der Bestimmung des Gesamtgehaltes an organischem Kohlenstoff (TOC) in einem Reaktionsgefäß zu verbrennen und das Verbrennungsgas geeigneten Detektoren zum Nachweis von Verbindungen zuzuführen, deren Erfassung einen Rückschluss auf den Gehalt an organischem Kohlenstoff der wässrigen Lösung erlaubt. Ein Verfahren zur TOC-Bestimmung ist aus der EP 0 887 643 A1 bekannt. Bei diesem Verfahren wird die Probe zunächst von einer Ausgangstemperatur unterhalb der Siedetemperatur des Wassers auf eine Verdampfungstemperatur und in einem zweiten Schritt auf eine wesentlich höhere Verbrennungstemperatur, bevorzugt im Bereich zwischen 800 und 1000 °C gebracht. Aus der DE 199 23 139 A1 sind ein Verfahren und eine Vorrichtung zum Aufschluss einer wässrigen Lösung zur Kohlenstoffgehaltsbestimmung bekannt, bei denen der Aufschluss eine katalysatorfreie Verbrennung bei einer Temperatur oberhalb von 1000 °C, speziell oberhalb von 1200 °C, einschließt.

Ein weiterer gebräuchlicher Parameter zur Quantifizierung der organischen Abwasserbelastung ist der totale Sauerstoffbedarf (TSB), dessen Bestimmung eine thermische Oxidation durch Verbrennung der Probe in einem Hochtemperaturreaktor einschließt. In der JP-B-977-26111 wird eine kombinierte TOC- und TOD-Messung beschrieben, bei der die Probe in einer Verbrennungskammer aufgeschlossen wird.

Bei Analyseverfahren und entsprechenden Geräten der genannten Art ist es für die Gewinnung aussagekräftiger Ergebnisse vielfach von Vorteil, neben den primären (beispielsweise am Einlauf einer Abwasserreinigungsanlage entnommenen) Proben verdünnte Proben zu untersuchen. Diese werden üblicherweise durch Vermischen einer kleinen Menge der primären Probelösung mit destilliertem Wasser in einer peripheren Mischeinrichtung der Messanordnung erzeugt und auch dort gespeichert.

Aus den Druckschriften US 4968485 A und EP 1022564 A2 sind Verfahren und Vorrichtungen bekannt, die in Bezug zum Gegenstand der vorliegenden Anmeldung stehen und jeweils bestimmte Merkmale des beanspruchten Probeanalysegerätes bzw. des beanspruchten Analyseverfahrens aufweisen. Speziell ist aus der US 4968485 A ein Verfahren mit einer Vorrichtung zur Vorbereitung der Analyse von Wasserproben bekannt, bei denen eine Einspritzeinheit mit einem Einspritzanschluss einer Verbrennungseinheit zur Verbrennung der Wasserproben zusammenwirkt und auch ein Probewasserbehälter vorhanden ist.

Der Erfindung liegt die Aufgabe zugrunde, ein verbessertes Probenanalysegerät und ein verbessertes Verfahren der eingangs genannten Art bereitzustellen, bei denen insbesondere die Handhabung verdünnter Proben erleichtert und damit der Bedienungsaufwand und die Betriebskosten verringert werden können.

Diese Aufgabe wird in ihrem Vorrichtungsaspekt durch ein Probenanalysegerät mit den Merkmalen des Anspruchs 1 und in ihrem Verfahrensaspekt durch ein Verfahren mit den Merkmalen des Anspruchs 11 gelöst. Zweckmäßige Fortbildungen des Erfindungsgedankens sind Gegenstand der jeweiligen abhängigen Ansprüche.

Die Erfindung schließt den Gedanken ein, die Bereitstellung verdünnter Proben unmittelbar in das Analyseverfahren einzubetten und entsprechende technische Einrichtungen in das eigentliche Probenanalysegerät zu integrieren. Sie schließt weiter den Gedanken ein, die Herstellung bzw. Zubereitung der verdünnten Probelösung, aus der eine verdünnte Probe gezogen werden kann, in einem zusätzlichen Gefäß des Analysegerätes auszuführen, und zwar in dem gleichen Gefäß, in dem die verdünnte Probelösung dann auch für die Zeitdauer aufbewahrt wird, für die sie benötigt wird.

Des Weiteren schließt die Erfindung den Gedanken ein, zur Herstellung der verdünnten Probelösung die vorhandene Injektionsspritzeneinrichtung zu nutzen, mit der Proben aus der primären Probelösung in das Reaktionsgefäß überführt werden. Der besagten Injektionsspritzeneinrichtung wird also gemäß dieser Überlegung der Erfinder eine zusätzliche Funktion zugeordnet, womit sich gesonderte Mittel zum Zuführen abgemessener Mengen der primären Probelösung in das Gefäß zum Erzeugen der Verdünnten Probelösung erübrigen.

Letztlich ist unter Vorrichtungsaspekten vorgesehen, dass mindestens ein erstes und zweites Probelösungs-Speichergefäß vorgesehen sind, wobei das zweite Probelösungs-Speichergefäß zur Herstellung und Speicherung verdünnter Probelösung ausgebildet ist, und die Injektionsspritzeneinrichtung zum Eintragen von aus dem ersten Probelösungs-Speichergefäß aufgenommener (primärer) Probelösung wahlweise in das zweite Probelösungs-Speichergefäß zur Herstellung der verdünnten Probelösung ausgebildet ist.

Mit der Erfindung wird eine kompakte, übersichtlich aufgebaute und leicht zu steuernde Analyseanordnung bereitgestellt, mit der verdünnte Proben analysiert werden können. Es versteht sich, dass eine solche Anordnung gegenüber den bisher bekannten Anordnungen aus Analysegerät und Peripherieeinrichtungen Platz- und Kostenvorteile mit sich bringt. In ähnlicher Weise vorteilhaft ist das vorgeschlagene Verfahren.

In einer Ausführung der Erfindung ist das zweite Probelösungs-Speichergefäß mit einem steuerbaren Wasseranschluss zur Zuführung von destilliertem Wasser zur Verdünnung von Probelösung verbunden. Grundsätzlich kann destilliertes Wasser auch manuell in das zweite Probelösungs-Speichergefäß gebracht werden, etwa mittels einer Pipette, eines kleinen Wasserkännchens o.ä.; für Routine-Analysearbeiten wären solche Mittel aber in ihrem Fassungsvermögen unzureichend und in ihrer Handhabung zu umständlich.

Gemäß einer weiteren Ausführung weist das zweite Probelösungs-Speichergefäß ein Innengefäß zur Herstellung und Speicherung der verdünnten Probelösung auf, an dessen Wandung mindestens abschnittsweise ein Überlaufbereich zum Ableiten von überschüssiger verdünnter Probelösung vorgesehen ist. Bei dieser Ausführung ist es möglich, das Gesamtvolumen des Innengefäßes als Bezugsvolumen an destilliertem Wasser für die Herstellung der verdünnten Probelösung zu benutzen; zugleich ermöglicht das Vorhandensein eines Überlaufbereiches ein unkompliziertes Spülen des zweiten Speichergefäßes zwischen verschiedenen Betriebsabläufen mit unterschiedlichen Probelösungen.

In einer weiteren Ausführung der Erfindung ist im zweiten Probelösungs-Speichergefäß, insbesondere im Innengefäß, ein Rührer zum Umrühren der verdünnten Probelösung vorgesehen. Hierdurch lässt sich in sehr kurzer Zeit nach dem Befüllen des zweiten Speichergefäßes mit Wasser und einer definierten Menge primärer Probelösung eine vollständig durchmischte verdünnte Probelösung herstellen und deren Homogenität auch während einer längeren Betriebsphase und Betriebsunterbrechungen aufrechterhalten. In einer speziellen, vorteilhaften Ausgestaltung ist der Rührer als Magnetrührer mit kontaktlosem Antrieb ausgebildet.

In einer weiteren Ausführung ist dem zweiten Probelösungs-Speichergefäß, insbesondere dem Innengefäß, eine durch eine obere Stirnfläche in dieses hineinragendes Röhrchen zum Zuführen von destilliertem Wasser zugeordnet. Das Röhrchen ist insbesondere mit dem weiter oben erwähnten Wasseranschluss verbunden beziehungsweise realisiert diesen, zusammen mit einem steuerbaren Ventil, einer Pumpe o.ä.

In einer weiteren Ausführung weisen das erste und zweite Probelösungs-Speichergefäß jeweils eine mit einer Schraubkappe verschlossene obere Stirnfläche auf, in der ein Injektionsport zum Einführen einer Injektionsnadel der Injektionsspritzeneinrichtung vorgesehen ist. Grundsätzlich kann die obere Stirnfläche der Speichergefäße auch komplett offen bleiben, im rauen Routine-Analysebetrieb will man den Inhalt aber vor Verunreinigungen aus der Atmosphäre schützen und sieht deshalb eine Abdeckung vor. Diese könnte wiederum grundsätzlich durch ein mit der Injektionsnadel durchstoßbares Septum gebildet werden, auch ein Septum ist aber im Routinebetrieb mit sehr vielen Durchstoßvorgängen weniger geeignet als ein Verschluss mit vorgefertigter Öffnung (und zugleich Führung) für die Nadel der Injektionsspritzeneinrichtung. In einer Ausgestaltung ist vorgesehen, dass zugleich das dem zweiten Probelösungs-Speichergefäß zugeordnete Röhrchen den Schraubverschluss durchstößt und in diesem gehaltert.

In einer weiteren Ausführung ist die Injektionsspritzeneinrichtung zur Aufnahme unterschiedlicher vorbestimmter Probemengen aus dem ersten Probelösungs-Speichergefäß zur Herstellung von verdünnten Probelösungen unterschiedlicher Konzentration steuerbar. Dies stellt eine Modifikation der Injektionsspritzeneinrichtung gattungsgemäßer Analysegeräte insofern dar, als jene typischerweise nur einen einzigen Wert an Probevolumen aus dem Probelösungs-Speichergefäß entnehmen und in das Reaktionsgefäß eintragen. In praktisch relevanten Ausführungen ist das vorgeschlagene Probenanalysegerät ausgebildet als Wasser- oder Abwasseranalysegerät zur Bestimmung des Gesamt-Kohlenstoffgehalts, TC, des Gesamtgehalts an anorganischem Kohlenstoff, TIC, des totalen Sauerstoffbedarfs, TSB, oder ähnlicher Parameter. Grundsätzlich ist die Anwendung der Erfindung aber nicht auf Geräte zur Bestimmung jener Parameter und auch nicht unbedingt auf Wasser- und Abwasseranalysegeräte beschränkt, sondern auch bei anderen Analysegeräten für flüssige Proben möglich, bei denen eine Probenverdünnung erforderlich ist oder erwünscht sein kann.

Unter Verfahrensaspekten zeichnet sich die Erfindung dadurch aus, dass geräteintern aus einer zugeführten primären Probelösung eine verdünnte sekundäre Probelösung hergestellt und gespeichert und wahlweise dem Reaktionsgefäß zugeführt wird.

Im erfindungsgemäßen Verfahren wird die verdünnte (sekundäre) Probelösung durch Zumischen einer definierten Menge primärer Probelösung zu einem definierten Volumen von destilliertem Wasser hergestellt. Dabei wird das Zumischen in einem separaten Probenspeichergefäß derart ausgeführt, dass dieses über eine Zufuhrleitung bis zu einem bestimmten Füllstand des Gefäßes oder bis zum Überlaufrand eines hierin vorgesehenen Innengefäßes mit destilliertem Wasser befüllt und anschließend mittels einer Injektionsspritze die abgemessene Menge primärer Probelösung eingespritzt wird. In einer weiteren Ausführung wird die verdünnte Probelösung mindestens zeitweise, insbesondere permanent, umgerührt. Die Vorteile dieser Ausgestaltung wurden bereits weiter oben unter Vorrichtungsaspekten erwähnt.

Vorteile und Zweckmäßigkeiten der Erfindung ergeben sich im Übrigen aus der nachfolgenden Beschreibung eines Ausführungsbeispiels anhand der Figuren. Von diesen Zeigen:
Fig. 1 eine synoptische schematische Darstellung eines Ausführungsbeispiels des erfindungsgemäßen Probenanalysegerätes und Verfahrens, in Art eines Fließschemas, und
Fig. 2 eine perspektivische Darstellung eines Probelösungs-Speichergefäßes zur Aufnahme einer verdünnten Probelösung als Ausführungsbeispiel.

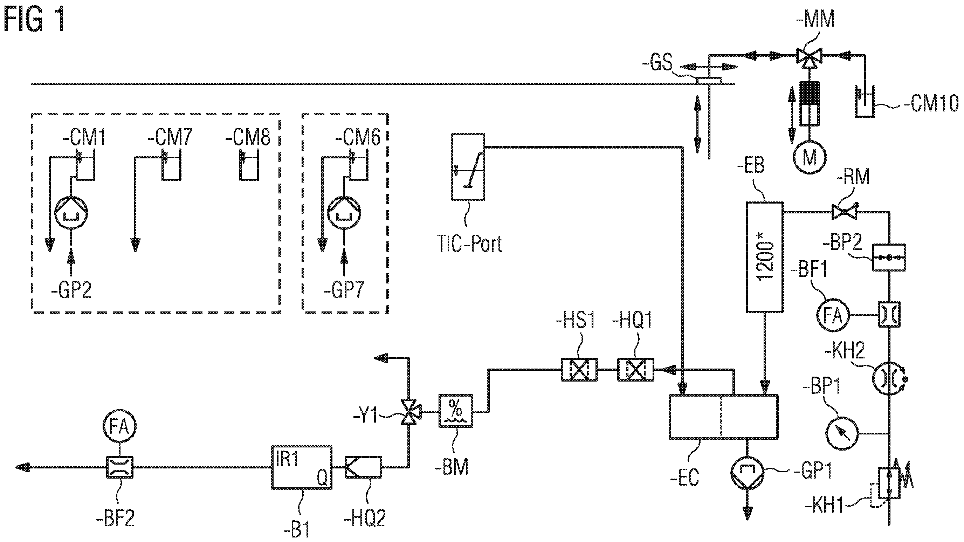

Fig. 1 zeigt schematisch die wesentlichen Komponenten eines beispielhaften Probenanalysegerätes gemäß der Erfindung und illustriert zugleich wesentliche Aspekte des erfindungsgemäßen Verfahrens. Geräte dieser Art - ohne die mit der Probenverdünnung und Speicherung und Verwendung verdünnter Proben befassten Teile und entsprechenden Verfahrensschritte - sind dem Fachmann an sich bekannt und wurden früher an anderer Stelle beschrieben, so etwa in der (unveröffentlichten) deutschen Patentanmeldung 10 2014 118 138.7 der Anmelderin. Nachfolgend werden daher vorrangig die mit der Erfindung im Zusammenhang stehenden Komponenten und Schritte erläutert.

Zentrale Komponenten des Probenanalysegerätes sind ein Reaktionsgefäß EB und eine Injektionsspritzeneinrichtung MM mit einer motorisch zwischen verschiedenen Gefäßen des Gerätes und dem Reaktionsgefäß EB verfahrbaren und motorisch betätigbaren Injektionsnadel GS. Eingangsseitig des Reaktionsgefäßes EB ist eine Reihe von Komponenten vorgesehen, die zur Bereitstellung eines geeignet zusammengesetzten und hinsichtlich Druck und Durchfluss gesteuerten Trägergasstromes zum Abtransport einer im Reaktionsgefäß aufgeschlossenen Probe aus dem Reaktionsgefäß dienen. Es handelt sich hierbei im Einzelnen um einen Druckminderer KH1, einen Druckmesser BP1, einen Durchflussregler KH2, einen Volumenstrommesser BFlund einen Drucksensor BP2. Unmittelbar eingangsseitig des Reaktionsgefäßes EB ist ein Rückschlagventil RM vorgesehen, welches zur Verhinderung eines Trägergas-Rückschlages beim Einspritzen einer Probe in das Reaktionsgefäß in Folge der damit schlagartig einsetzenden Druckerhöhung dient.

Ausgangsseitig des Reaktionsgefäßes EB befinden sich ein Gaskühler EC und verschiedene Filter, hier ein Quarzfilter HQ1, und ein Säurefilter HS1, sowie ein Feuchtigkeitssensor BM und ein Dreiwegeventil Y1. Dieses leitet die gekühlte und gefilterte Mischung aus Trägergas und aufgeschlossener Probe wahlweise zu weiteren Gerätekomponenten, hier zu einem Gasfilter HQ2, einem IR Detektor B1 zum CO2 Nachweis und einem Volumenstrommesser BF2. Dem Gaskühler sind eine Kondensatpumpe GP1 zur Ableitung von Kondensatwasser sowie eingangsseitig ein TIC-Port zugeordnet; siehe dazu weiter unten.

Das Probenanalysegerät enthält des Weiteren mehrere Gefäße zur geräteinternen Speicherung von für das Verfahren benötigten Flüssigkeiten. Es handelt sich hierbei um ein erstes Probelösungs-Speichergerät (hier auch bezeichnet als Probenvorlagegefäß) CM1 mit einem Zulauf, in dem eine Probelösungs-Förderpumpe GP2 angeordnet ist, ein Spüllösungs-Speichergefäß (hier auch kurz bezeichnet als Spülgefäß) CM7 und ein Kalibrierungslösungs-Speichergefäß CM8. Erfindungsgemäß ist außerdem ein zweites Probelösungs-Speichergefäß (auch bezeichnet als Verdünnungsgefäß) CM6 mit einem Zulauf vorgesehen, in dem eine Förderpumpe GP7 für destilliertes Wasser zur Herstellung der in diesem Speichergefäß zubereiteten und aufbewahrten verdünnten Probelösung zugeordnet ist.

Ein beispielhafter Ablauf zur Bereitstellung einer verdünnten Probe mit dem in Fig. 1 dargestellten Geräteaufbau ist wie folgt:
- Die dem ersten Probelösungs-Speichergefäß CM1 zugeordnete Förderpumpe GP2 füllt das erste Probelösungs-Speichergerät mit primärer (beispielsweise am Einlauf einer Abwasserreinigungsanlage entnommener) Probelösung. Bei Erreichung eines vorbestimmten Füllstandes oder auch Überlauf stoppt die Förderpumpe GP2.
- Die dem zweiten Probelösungs-Speichergefäß CM6 zugeordnete Förderpumpe GP7 füllt dieses Gefäß (oder spezieller dessen Innengefäß; siehe die Beschreibung eines beispielhaften zweiten Probelösungs-Speichergefäßes weiter unten) mit destilliertem Wasser. Wenn ein bestimmter Füllstand erreicht ist oder ein Überlauf aus dem erwähnten Innengefäß festzustellen ist, stoppt die Förderpumpe GP7.
- Die Injektionsspritzeneinrichtung MM wird so gesteuert/angetrieben, dass die Injektionsnadel GS zum ersten Probelösungs-Speichergefäß (Probenvorlagegefäß) CM1 fährt, dort eintaucht und ein vorbestimmtes Probevolumen der primären Probelösung entnimmt.
- Anschließend wird die Injektionsspritzeneinrichtung MM derart gesteuert/angetrieben, dass die Nadel GS zum zweiten Probelösungs-Speichergefäß (Probenverdünnungsgefäß) CM6 fährt, dort speziell in das Innengefäß, eintaucht und das aus dem Gefäß CM1 entnommene Probevolumen unterhalb des Wasserspiegels des dort stehenden destillierten Wassers (beispielhaft 60 % tiefer) einspritzt.
- Anschließend wird die Injektionsspritzenreinrichtung MM derart betätigt, dass die Nadel GS zum Spülgefäß CM7 fährt und dort gespült wird.
- Im Probenverdünnungsgefäß CM6 wird die dort eingespritzte Probemenge und das dort vorab eingefüllte destillierte Wasser mittels eines Rührers homogenisiert. Für nachfolgende Schritte wird eine vorbestimmte Zeitspanne des Homogenisierens abgewartet.

Bei einer anschließenden Messung des Gesamt-Kohlenstoffgehalts (TC), des Gesamt-Stickstoffgehalts (TN) oder des chemischen Sauerstoffbedarfs (COD) einer Wasser- oder Abwasserprobe werden folgende Schritte ausgeführt:
- Die Injektionsnadel GS wird zum Probenverdünnungsgefäß CM6 gefahren und zieht dort ein vorbestimmtes Probevolumen auf.
- Anschließend fährt die Injektionsnadel GS zum Reaktionsgefäß EB und trägt die aus dem Probenverdünnungsgefäß gezogene Probe dort ein.
- Anschließend fährt die Injektionsnadel GS zum Spülgefäß CM7 und wird dort gespült.
- Das durch thermische Reaktion (Verbrennung) im Reaktionsgefäß EB gewonnene Reaktionsprodukt wird mittels des eingangsseitig zugeführten Trägergases durch den Gaskühler EC und verschiedene Filter zu verschiedenen Detektoren transportiert.
- Die Detektoren ermitteln gemäß ihren Detektorspezifikationen Roh-Messwerte, die durch geeignete Software auf an sich bekannte Weise in Analysewerte der Probe umgerechnet werden.

Bei einer Messung des Gehalts an anorganischem Kohlenstoff (TIC), wo der anorganische Kohlenstoff in der Probe durch Säurevorlage im TIC-Port ins Trägergas überführt wird, ist ein beispielhafter Ablauf wie folgt:
- Die Injektionsnadel GS wird zum Probenverdünnungsgefäß CM6 gefahren und zieht dort ein vorbestimmtes Probevolumen auf.
- Die Injektionsnadel GS fährt anschließend zum TIC-Port und spritzt die aus dem Probenverdünnungsgefäß CM6 gezogene Probe dort in eine spezielle Lösung, einen sog. Stripper, ein. Durch die saure Wirkung der in dem Stripper enthaltenen Phosphorsäure wird das gebundene CO2 freigesetzt und mit dem Trägergas zu dem CO2-Detektor B1 geleitet.
- Anschließend wird die Injektionsspritzenreinrichtung MM derart betätigt, dass die Nadel GS zum Spülgefäß CM7 fährt und dort gespült wird.

Zur Bestimmung des Gehalts an organischem Kohlenstoff (TOC) einer Probe wird durch geeignete Software eine Differenzbildung aus den nach obigem ermittelten Werten des TC und des TIC vorgenommen.

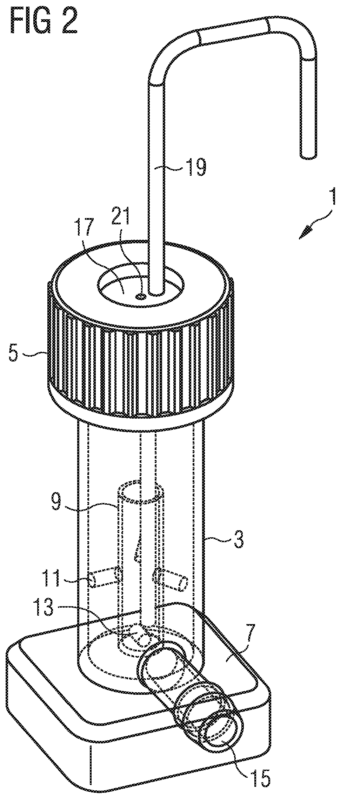

Fig. 2 zeigt in perspektivischer Ansicht eine Ausführung des zweiten Probelösungs-Speichergefäßes (Probenverdünnungsgefäßes), das in Fig. 1 als CM6 bezeichnet wurde und in Fig. 2 mit der Ziffer 1 bezeichnet ist. Hauptteile des Probenverdünnungsgefäßes 1 sind ein zylindrischer Glaskörper (Außengefäß) 3, ein Kunststoff-Schraubverschluss 5 und eine Rührer-Antriebsplatte 7.

Im Außengefäß 3, das in seiner Geometrie beispielhaft auf diejenige der weiter oben erwähnten anderen Speichergefäße abgestimmt ist, ist ein Innengefäß 9 mit wesentlich geringerem Durchmesser und geringerer Höhe zentrisch durch Glas-Abstandshalter 11 fixiert. Nahe dem Boden des Innengefäßes 9 liegt horizontal ein kleiner Rührstab 13, der aus magnetischem Material gebildet ist und durch eine entsprechende Antriebseinheit in der Rührer-Antriebsplatte 7 in Drehung in einer horizontalen Ebene versetzt werden kann. Nahe seinem Boden hat das Verdünnungsgefäß 3 einen Ablauf 15.

Der Schraubverschluss 5 hält eine kreisscheibenförmige Verschlussplatte 17 des Probenverdünnungsgefäßes 3, in der eine (nicht gesondert bezeichnete) Öffnung für ein Röhrchen 17 zur Zuführung von destilliertem Wasser in das Innengefäß 9 und ein Injektionsport 21 zum Führen der Injektionsnadel GS (Fig. 1) beim Einspritzen von Probe in das Probenverdünnungsgefäß vorgesehen sind.

Wie weiter oben bereits erwähnt, wird verdünnte Probelösung im Probenverdünnungsgefäß 1 zubereitet, indem das Innengefäß 9 über das Röhrchen 19 zunächst mit destilliertem Wasser befüllt wird, bis es vollständig gefüllt ist und ein Überlauf über dessen oberen Rand in den Ringraum zum Außengefäß 3 und durch den Überlauf 15 zu verzeichnen ist. Dann wird die Zufuhr von destilliertem Wasser gestoppt, und in einem Folgeschritt wird die Injektionsnadel GS zum Probenverdünnungsgefäß 1 verfahren und in den Injektionsport 21 so eingeführt, dass ihr Ende bis tief in das Innengefäß 9 reicht. In diesem Zustand wird der Kolben der Injektionsspritze aktiviert und das Vorab im Probenvorlagegefäß aufgenommene Probenvolumen in das Innengefäß eingespritzt. Hierdurch wird eine verdünnte Probelösung mit vorbestimmtem Verdünnungsgrad zubereitet. Hierbei läuft eine dem Probenvolumen entsprechende Menge an destilliertem Wasser über und verlässt das Außengefäß 3 wiederum durch den Überlauf 15. Zwischen verschiedenen Messreihen mit unterschiedlicher Probenverdünnung wird das Innengefäß jeweils mit über das Röhrchen 19 zugeführtem destilliertem Wasser gespült.

## Patentansprüche

1. Probenanalysegerät zur Analyse einer Probelösung, insbesondere von verunreinigtem Wasser oder von Abwasser, mit einem Reaktionsgefäß (EB) zum thermischen Aufschluss einer abgemessenen Probe der zu analysierenden Probelösung, wobei das Reaktionsgefäß (EB) einen Injektionsport zum Eintragen der Probe in das Reaktionsgefäß aufweist, mindestens einem Probelösungs-Speichergefäß (CM1) zur geräteinternen Speicherung von Probelösung und einer zwischen dem Probelösungs-Speichergefäß (CM1) und dem Reaktionsgefäß (EB) verfahrbaren und zum Aufnehmen der Probe aus dem Probelösungs-Speichergefäß (CM1) und zum Eintragen der Probe in den Injektionsport des Reaktionsgefäßes (EB) steuerbaren Injektionsspritzeneinrichtung (MM), **dadurch gekennzeichnet, dass** mindestens ein erstes und zweites Probelösungs-Speichergefäß vorgesehen sind, wobei das zweite Probelösungs-Speichergefäß (CM6) zur Herstellung und Speicherung verdünnter Probelösung ausgebildet ist, und die Injektionsspritzeneinrichtung (MM) zum Eintragen von aus dem ersten Probelösungs-Speichergefäß (CM1) aufgenommener Probelösung wahlweise in das zweite Probelösungs-Speichergefäß (CM6) zur Herstellung der verdünnten Probelösung ausgebildet ist.

2. Probenanalysegerät nach Anspruch 1, wobei das zweite Probelösungs-Speichergefäß (CM6) mit einem steuerbaren Wasseranschluss zur Zuführung von destilliertem Wasser zur Verdünnung von Probelösung verbunden ist.

3. Probenanalysegerät nach Anspruch 1 oder 2, wobei das zweite Probelösungs-Speichergefäß (CM6) ein Innengefäß (9) zur Herstellung und Speicherung der verdünnten Probelösung aufweist, an dessen Wandung mindestens abschnittsweise ein Überlaufbereich (15) zum Ableiten von überschüssiger verdünnter Probelösung vorgesehen ist.

4. Probenanalysegerät nach einem der vorangehenden Ansprüche, wobei im zweiten Probelösungs-Speichergefäß, insbesondere im Innengefäß, ein Rührer (13) zum Umrühren der verdünnten Probelösung vorgesehen ist.

5. Probenanalysegerät nach Anspruch 4, wobei der Rührer (13) als Magnetrührer mit kontaktlosem Antrieb ausgebildet ist.

6. Probenanalysegerät nach einem der Ansprüche 2 - 5, wobei dem zweiten Probelösungs-Speichergefäß, insbesondere dem Innengefäß, eine durch eine obere oder untere Stirnfläche in dieses hineinragendes Röhrchen (19) zum Zuführen von destilliertem Wasser zugeordnet ist

7. Probenanalysegerät nach einem der vorangehenden Ansprüche, wobei das erste und zweite Probelösungs-Speichergefäß jeweils eine mit einer Schraubkappe (5) verschlossene obere Stirnfläche aufweisen, in der ein Injektionsport zum Einführen einer Injektionsnadel (GS) der Injektionsspritzeneinrichtung (MM) vorgesehen ist,

8. Probenanalysegerät nach Anspruch 6 und 7, wobei das dem zweiten Probelösungs-Speichergefäß (CM1, CM6) zugeordnete Röhrchen 19) die Schraubkappe (5) durchstößt und in diesem gehaltert ist.

9. Probenanalysegerät nach einem der vorangehenden Ansprüche, wobei die Injektionsspritzeneinrichtung (MM) zur Aufnahme unterschiedlicher vorbestimmter Probemengen aus dem ersten Probelösungs-Speichergefäß (CM1) zur Herstellung von verdünnten Probelösungen unterschiedlicher Konzentration steuerbar ist.

10. Probenanalysegerät nach einem der vorangehenden Ansprüche, ausgebildet als Wasser- oder Abwasseranalysegerät zur Bestimmung des Gesamt-Kohlenstoffgehalts, TC, des Gesamtgehalts an anorganischem Kohlenstoff, TIC, des totalen Sauerstoffbedarfs, TSB, oder ähnlicher Parameter.

11. Verfahren zur Analyse einer Probelösung, insbesondere von verunreinigtem Wasser oder von Abwasser, wobei Probelösung geräteintern in mindestens einem Probelösungs-Speichergefäß (CM1) gespeichert wird und eine mengenmäßig vorbestimmte Probe der Probelösung in einem Reaktionsgefäß (EB) thermisch aufgeschlossen wird und die Aufschlussprodukte einer Detektionseinrichtung zur quantitativen Detektion von Elementen, insbesondere Kohlenstoff, Stickstoff oder Phosphor, zugeführt werden,
**dadurch gekennzeichnet, dass** geräteintern aus einer zugeführten primären Probelösung eine verdünnte sekundäre Probelösung hergestellt und gespeichert und wahlweise dem Reaktionsgefäß (EB) zugeführt wird, wobei die verdünnte sekundäre Probelösung durch Zumischen einer definierten Menge primärer Probelösung zu einem definierten Volumen von destilliertem Wasser hergestellt wird und wobei das Zumischen in einem separaten Probenspeichergefäß (CM6) derart ausgeführt wird, dass dieses über eine Zufuhrleitung bis zu einem bestimmten Füllstand des Gefäßes oder bis zum Überlaufrand eines hierin vorgesehenen Innengefäßes (9) mit destilliertem Wasser befüllt und anschließend mittels einer Injektionsspritze (MM) die abgemessene Menge primärer Probelösung eingespritzt wird.

12. Verfahren nach Anspruch 11, wobei die verdünnte Probelösung mindestens zeitweise, insbesondere permanent, umgerührt wird.

13. Verfahren nach Anspruch 11 oder 12, angewandt zur Bestimmung des Gesamt-Kohlenstoffgehalts, TC, des Gesamtgehalts an anorganischem Kohlenstoff, TIC, des totalen Sauerstoffbedarfs, TSB, oder ähnlicher Parameter.

## Claims

1. A sample analysis device for analyzing a sample solution, in particular of contaminated water or waste water, having a reaction vessel (EB) for thermal digestion of a weight-measured sample of the sample solution to be analyzed, wherein the reaction vessel (EB) includes an injection port for introducing the sample into the reaction vessel, at least one sample solution storage vessel (CM1) for storing the sample solution internally in the device, and a controllable injection syringe device (MM) that is movable between the sample solution storage vessel (CM1) and the reaction vessel (EB) and adapted to take up the sample from the sample solution storage vessel (CM1) and to introduce the sample into the injection port of the reaction vessel (EB),
**characterized in that**
at least one first and second sample solution storage vessels are provided, wherein the second sample solution storage vessel (CM6) is formed for preparing and storing diluted sample solution, and the injection syringe device (MM) is formed for introducing sample solution taken up from the first sample solution storage vessel (CM1) optionally into the second sample solution storage vessel (CM6) for preparing the diluted sample solution.

2. The sample analysis device according to claim 1, wherein the second sample solution storage vessel (CM6) is connected to a controllable water connection for introducing distilled water so as to dilute the sample solution.

3. The sample analysis device according to claim 1 or 2, wherein the second sample solution storage vessel (CM6) includes an internal vessel (9) for preparing and storing the diluted sample solution, at the wall of which, at least in sections, an overflow area (15) is provided for discharging excessive diluted sample solution.

4. The sample analysis device according to any one of the preceding claims, wherein a stirrer (13) is provided within the second sample solution storage vessel, in particular within the internal vessel, for stirring the diluted sample solution.

5. The sample analysis device according to claim 4, wherein the stirrer (13) is formed as a magnetic stirrer with a contactless drive.

6. The sample analysis device according to any one of claims 2 to 5, wherein the second sample solution storage vessel, in particular the internal vessel, has a small tube (19) assigned projecting into it through an upper or lower end surface for supplying distilled water.

7. The sample analysis device according to any one of the preceding claims, wherein the first and second sample solution storage vessels each include an upper end surface that is closed by a screw cap, and in which an injection port is provided for introducing an injection needle (GS) of the injection syringe device (MM).

8. The sample analysis device according to claims 6 and 7, wherein the small tube (19) assigned to the second sample solution storage vessel (CM1, CM6) penetrates the screw cap (5) and is supported therein.

9. The sample analysis device according to any one of the preceding claims, wherein the injection syringe device (MM) is controllable for taking up different predetermined sample quantities from the first sample solution storage vessel (CM1) so as to prepare diluted sample solutions of different concentrations.

10. The sample analysis device according to any one of the preceding claims, formed as a water or waste water analysis device for determining the total carbon content (TC), the total content of inorganic carbon (TIC), the total oxygen demand (TSB) or similar parameters.

11. A method for analyzing a sample solution, in particular of contaminated water or waste water, wherein the sample solution is stored internally in a device in at least one sample solution storage vessel (CM1), and a quantitatively predetermined sample of the sample solution is thermally digested in a reaction vessel (EB), and the digestion products are supplied to a detection device for quantitatively detecting elements, in particular carbon, nitrogen or phosphor,
**characterized in that**
internally in the device, a diluted secondary sample solution from a supplied primary sample solution is prepared and stored and is optionally supplied to the reaction vessel (EB), wherein the diluted secondary sample solution is prepared by mixing a defined quantity of primary sample solution into a defined volume of distilled water, and wherein the mixing is performed in a separate sample storage vessel (CM6) in such a way that the latter is filled with distilled water via a feed line up to a determined filling level of the vessel or up to an overflow edge of an internal vessel (9) provided there, and subsequently the weight-measured quantity of primary sample solution is injected by means of an injection syringe (MM).

12. The method according to claim 11, wherein the diluted sample solution is stirred at least temporarily, in particular permanently.

13. The method according to claim 11 or 12, applied for determining the total carbon content (TC), the total content of inorganic carbon (TIC), the total oxygen demand (TSB) or similar parameters.

## Revendications

1. Appareil d'analyse d'échantillon pour l'analyse d'une solution d'échantillon, en particulier d'eau souillée ou d'eaux usées, comprenant un récipient de réaction (EB) pour la minéralisation thermique d'un échantillon dosé de la solution d'échantillon à analyser, sachant que le récipient de réaction (EB) présente un orifice d'injection pour l'introduction de l'échantillon dans le récipient de réaction, au moins un récipient de stockage de solution d'échantillon (CM1) pour le stockage interne à l'appareil de solution d'échantillon et un dispositif de seringue d'injection (MM) translatable entre le récipient de stockage de solution d'échantillon (CM1) et le récipient de réaction (EB) et commandable pour la réception de l'échantillon depuis le récipient de stockage de solution d'échantillon (CM1) et pour l'introduction de l'échantillon dans l'orifice d'injection du récipient de réaction (EB),
**caractérisé en ce qu'**au moins un premier et un deuxième récipient de stockage de solution d'échantillon sont prévus, sachant que le deuxième récipient de stockage de solution d'échantillon (CM6) est constitué pour la fabrication et le stockage de solution d'échantillon diluée, et le dispositif de seringue d'injection (MM) est constitué pour l'introduction de solution d'échantillon réceptionnée depuis le premier récipient de stockage de solution d'échantillon (CM1) facultativement dans le deuxième récipient de stockage de solution d'échantillon (CM6) pour la fabrication de la solution d'échantillon diluée.

2. Appareil d'analyse d'échantillon selon la revendication 1, sachant que le deuxième récipient de stockage de solution d'échantillon (CM6) est relié à un raccord d'eau commandable pour l'amenée d'eau distillée pour la dilution de solution d'échantillon.

3. Appareil d'analyse d'échantillon selon la revendication 1 ou 2, sachant que le deuxième récipient de stockage de solution d'échantillon (CM6) présente un récipient intérieur (9) pour la fabrication et le stockage de la solution d'échantillon diluée, à la paroi duquel une zone de débordement (15) est prévue au moins par section pour l'évacuation de solution d'échantillon diluée excédentaire.

4. Appareil d'analyse d'échantillon selon l'une des revendications précédentes, sachant qu'un agitateur (13) est prévu dans le deuxième récipient de stockage de solution d'échantillon, en particulier dans le récipient intérieur, pour l'agitation de la solution d'échantillon diluée.

5. Appareil d'analyse d'échantillon selon la revendication 4, sachant que l'agitateur (13) est constitué comme agitateur magnétique à entraînement sans contact.

6. Appareil d'analyse d'échantillon selon l'une des revendications 2 à 5, sachant qu'au deuxième récipient de stockage de solution d'échantillon, en particulier au récipient intérieur, est associé un petit tube (19) dépassant dans celui-ci à travers une face frontale supérieure ou intérieure pour l'amenée d'eau distillée.

7. Appareil d'analyse d'échantillon selon l'une des revendications précédentes, sachant que le premier et le deuxième récipient de stockage de solution d'échantillon présentent respectivement une face frontale supérieure obturée par un capuchon vissé (5), dans laquelle un orifice d'injection est prévu pour l'introduction d'une aiguille d'injection (GS) du dispositif de seringue d'injection (MM).

8. Appareil d'analyse d'échantillon selon la revendication 6 ou 7, sachant que le petit tube (19) associé au deuxième récipient de stockage de solution d'échantillon (CM1, CM6) transperce le capuchon vissé (5) et est maintenu dans celui-ci.

9. Appareil d'analyse d'échantillon selon l'une des revendications précédentes, sachant que le dispositif de seringue d'injection (MM) est commandable pour la réception de différentes quantités d'échantillon prédéterminées depuis le premier récipient de stockage de solution d'échantillon (CM1) pour la fabrication de solutions d'échantillon diluées de concentration différente.

10. Appareil d'analyse d'échantillon selon l'une des revendications précédentes, constitué comme appareil d'analyse d'eau ou d'eaux usées pour la détermination de la teneur totale en carbone (TC), de la teneur totale en carbone inorganique (TIC), du besoin total en oxygène (TSB), ou de paramètres similaires.

11. Procédé d'analyse d'une solution d'échantillon, en particulier d'eau souillée ou d'eaux usées, sachant qu'une solution d'échantillon est stockée à l'intérieur de l'appareil dans au moins un récipient de stockage de solution d'échantillon (CM1) et un échantillon de la solution d'échantillon, prédéterminé en termes de quantité, est minéralisé thermiquement dans un récipient de réaction (EB) et les produits de minéralisation sont amenés à un dispositif de détection pour la détection quantitative d'éléments, en particulier de carbone, d'azote ou de phosphore,
**caractérisé en ce qu'**une solution d'échantillon secondaire diluée est fabriquée et stockée à l'intérieur de l'appareil à partir d'une solution d'échantillon primaire amenée et est amenée facultativement au récipient de réaction (EB), sachant que la solution d'échantillon secondaire diluée est fabriquée par adjonction d'une quantité définie de solution d'échantillon primaire à un volume défini d'eau distillée et sachant que l'adjonction est exécutée dans un récipient de stockage d'échantillon (CM6) séparé de telle manière que celui-ci soit rempli d'eau distillée via une conduite d'amenée jusqu'à un niveau de remplissage déterminé du récipient ou jusqu'au bord de débordement d'un récipient intérieur (9) prévu dans celui-ci et la quantité dosée de solution d'échantillon primaire soit ensuite injectée moyennant une seringue d'injection (MM).

12. Procédé selon la revendication 11, sachant que la solution d'échantillon diluée est agitée au moins par intermittence, en particulier en permanence.

13. Procédé selon la revendication 11 ou 12, appliqué à la détermination de la teneur totale en carbone (TC), de la teneur totale en carbone inorganique (TIC), du besoin total en oxygène (TSB) ou de paramètres similaires.
